# FASCICULE DE BREVET EUROPEEN

(11) **EP 2 887 878 B1**
(45) Date de publication et mention de la délivrance du brevet: **02.11.2016**
(21) Numéro de dépôt: 13762216.3
(22) Date de dépôt: 23.07.2013
(51) Int. Cl.: A61B 8/00, G10K 11/35, G01S 15/89

(54) **DISPOSITIF D'ÉCHOGRAPHIE TÉLÉCOMMANDÉ**
FERNGESTEUERTE ULTRASCHALLVORRICHTUNG
REMOTE-CONTROLLED ULTRASOUND DEVICE

(30) Priorité: 24.07.2012 FR 1202093
(43) Date de publication de la demande: 01.07.2015
(73) Titulaire: Advanced Echo Technology, 41310 Huisseau-en-Beauce (FR)
(72) Inventeur: LEFEBVRE, Eric, F-41310 Huisseau-en-Beauce (FR); CHARRON, Gwenaël, F-41100 Naveil (FR)
(74) Mandataire: Thibon, Norbert
(86) Numéro de dépôt international: PCT/IB2013/001598
(87) Numéro de publication internationale: WO 2014/016663

(56) Documents cités:
- EP-A1- 0 327 459
- US-A1- 2005 154 431
- US-A1- 2009 036 780
- US-A1- 2011 077 557

## Description

La présente invention concerne un dispositif d'échographie télécommandé. Ce dispositif s'applique notamment dans le domaine médical pour la pratique d'examens échographiques à distance, connus sous la notion de télé-échographie.

La télé-échographie concerne un examen d'échographie réalisé à distance. Dans le cas de la télé-échographie médicale qui sera décrit de façon particulière par la suite, cet examen à distance est réalisé sur un patient présent dans un site patient isolé où aucun médecin qualifié n'est présent. Le principe consiste à faire commander par un médecin qualifié depuis un site d'expertise une sonde d'échographie réelle, embarquée sur le site patient, et appliquée sur le patient par un opérateur qui n'a pas besoin de qualification médicale, ou par le patient lui-même. Les mouvements de la main du médecin, qui commande un « joystick » formant une sonde fictive, sont retransmis au niveau de la sonde d'échographie réelle présente en bout d'un robot. Le robot reproduit les mouvements imprimés par le médecin sensiblement en temps réel et le médecin reçoit sur un moniteur de' contrôle l'image captée par la sonde réelle.

Au titre des connaissances de l'état de la technique, on pourra évoquer les travaux du Professeur Philippe Arbeille, connu pour être un éminent spécialiste de la télé-échographie. La mise au point du dispositif d'échographie selon l'invention tire profit de ses travaux variés sur le sujet.

Par ailleurs, le document US 2011/077557 A1 décrit un dispositif médical à ultrasons comportant deux compartiments séparés de manière étanche, l'un comprenant des transducteurs et l'autre des moyens d'entraînement de ces transducteurs, un bras de commande de transducteurs traversant la séparation.

Dans ce contexte, l'invention vise à proposer un dispositif d'échographie télécommandé adapté à recevoir des instructions de déplacement de moyens capteurs en provenance d'un module de commande qui calcule les instructions en fonction du déplacement à distance d'une sonde fictive. Selon des caractéristiques essentielles de l'invention, le dispositif comporte une enveloppe d'enceinte à l'intérieur de laquelle sont disposés un compartiment étanche et un compartiment de commande séparés par une cloison transversale. L'enveloppe d'enceinte est destinée à être appliquée contre le corps du patient, et le dispositif est orienté de telle sorte que c'est le compartiment étanche qui est en regard du patient. Ce compartiment étanche est rempli d'un milieu permettant la propagation d'ondes ultrasonores, par exemple un bain d'huile, et la cloison assure que l'huile reste confinée à l'intérieur de ce compartiment étanche, sans se déverser à l'intérieur du compartiment de commande. Des moyens capteurs sont disposés dans le compartiment étanche pour prendre des images du corps du patient et ils sont immergés dans le milieu pour favoriser la propagation des ondes par les moyens capteurs, afin de recueillir les données nécessaires à la réalisation d'images échographiques. L'enveloppe d'enceinte présente au niveau du compartiment étanche une paroi transparente aux ultrasons. L'ensemble du compartiment étanche peut être réalisé en un tel matériau ou bien seule une fenêtre de visée de taille adéquate peut être réalisée. Les moyens capteurs sont déplacés au sein du compartiment étanche par l'action de moyens d'entraînement qui agissent directement sur une tige support à l'extrémité de laquelle sont montés les moyens capteurs. Les moyens capteurs et les moyens d'entraînement sont logés les uns et les autres respectivement dans une des parties fonctionnelles séparées par la cloison de sorte que pour les relier, la tige support traverse la cloison disposée entre le compartiment de commande et le compartiment étanche. On comprend que la tige support traverse la cloison de manière étanche pour s'assurer que le milieu dont est rempli le compartiment étanche ne s'évacue pas. Avantageusement, par cette conception, on peut générer un déplacement des moyens capteurs à l'intérieur du compartiment étanche tout en conservant fixe l'enveloppe d'enceinte.

Le dispositif selon l'invention permet ainsi de réaliser l'opération de télé-échographie sans qu'il y ait de mouvements parasites sur le ventre du patient, c'est à dire sans bouger l'enveloppe d'enceinte à son point de contact avec la peau du patient. Il n'y a pas de contact direct entre la peau et les moyens capteurs, qui seuls bougent.

Le fait d'avoir une enveloppe d'enceinte qui entoure la totalité des composants de la sonde d'échographie réelle permet un maniement simplifié par l'opérateur de sorte que l'on peut réaliser ces opérations dans des zones du corps peu accessibles.

Le milieu propagateur d'onde dans lequel sont immergés les moyens capteurs, qui peut prendre la forme d'un bain d'huile, permet de s'assurer de la bonne acquisition des images quelle que soit la position des moyens capteurs mobiles dans l'enveloppe d'enceinte fixe, et la cloison permet de s'assurer que l'huile ne se déverse pas hors du compartiment étanche, ce qui aurait pour effet à la fois de gêner les déplacements des moyens d'entraînement et de faire entrer de l'air qui gênerait la propagation d'ondes dans le compartiment étanche.

Selon différentes caractéristiques avantageuses de l'invention, on s'efforce de limiter le frottement sur la cloison dû au mouvement de la tige support qui entraîne en déplacement les moyens capteurs en fonction des déplacements des moyens d'entraînement.

D'une part, on commande le déplacement de la tige support de telle sorte que l'on fige la position du point de passage de la tige à travers la cloison, au centre de celle-ci. La tige support se déplace en basculant par rapport à ce point fixe central et les moyens capteurs disposés en bout de tige sont adaptés à se déplacer dans le compartiment étanche de façon pendulaire par rapport à ce point fixe disposé au centre de la cloison.

D'autre part, on peut prévoir de rapporter un tube au centre de la cloison, avec un joint d'étanchéité disposé autour du tube et plaqué contre la cloison, et de faire passer la tige à l'intérieur de ce tube. La tige est montée libre en rotation à l'intérieur du tube et les dimensions du tube et de la tige sont telles que les deux extrémités de la tige dépassent du tube, l'une pour porter les moyens capteurs et l'autre pour être entraîné en rotation propre par un actionneur. Il convient de prévoir les dimensions radiales telles que la tige peut tourner à l'intérieur du tube sans que de l'huile puisse s'écouler entre la tige et le tube. Ainsi, on peut réaliser la rotation propre de la tige sans que le tube ne tourne. On permet dans tous les cas de limiter les contraintes sur la cloison et d'éviter son déchirement.

Selon une caractéristique de l'invention, le tube présente à son extrémité opposée aux moyens capteurs une bague de guidage pour les moyens d'entraînement, et cette bague de guidage sert également de support pour l'actionneur en prise directe sur la tige pour commander la rotation propre de la tige à l'intérieur du tube. Il est dès lors plus facile de calculer les instructions pour chacun des actionneurs des moyens d'entraînement et de la tige puisque ceux-ci interagissent directement avec la même pièce.

Selon d'autres caractéristiques de l'invention, le compartiment étanche présente une forme hémisphérique avec la cloison pour base et les moyens capteurs présentent un rayon de courbure égal au rayon de courbure du compartiment étanche, de manière à ce que les moyens capteurs puissent se déplacer le long de la surface du compartiment étanche. Ainsi, on s'assure par ce déplacement surfacique que le jeu est constant entre le compartiment étanche et le capteur, ce jeu de fonctionnement étant déterminé pour que les moyens capteurs soient au plus proche du compartiment étanche sans contact. Le fait que le compartiment étanche soit rempli d'un milieu adapté à la propagation des ondes par les moyens capteurs.

On comprend en outre que le diamètre du compartiment étanche pourra être aussi petit que le permet la miniaturisation du capteur tout en respectant cette équivalence des rayons de courbure. Dès lors, on pourra proposer selon l'invention un dispositif d'échographie télécommandé plus compact, avec un compartiment de commande qui sera d'une largeur équivalente à celle du compartiment étanche. On pourra ainsi plus facilement appliquer ce dispositif pour des appareillages de télé-échographie embarqués dans des navettes spatiales par exemple.

Le dispositif d'échographie selon l'invention est adapté à recevoir d'une part des instructions de déplacement des moyens capteurs dans le compartiment étanche qui proviennent d'un module de commande associé au dispositif et d'autre part des informations d'activation des transducteurs piezoélectriques composant les moyens capteurs, qui proviennent également de ce module de commande.

Ainsi, on peut jouer à la fois sur le déplacement mécanique des moyens capteurs et sur le choix des cristaux à activer pour réaliser telle ou telle observation par les moyens capteurs avec une enveloppe d'enceinte qui reste fixe en place sur le corps du patient. Il convient d'implémenter le module de commande avec un logiciel adapté à transformer par le calcul les données du déplacement à distance d'une sonde fictive en des instructions de déplacement des moyens capteurs pour qu'ils puissent récolter des images échographiques similaires à celles qu'auraient obtenues le médecin qualifié s'il avait été sur place.

Selon différentes caractéristiques que l'on retrouve combinées dans un mode de réalisation présenté ci-après, les moyens d'entraînement comportent deux arceaux disposés perpendiculairement et montés avec un degré de liberté en rotation sur une platine support sur laquelle est-fixée l'enveloppe d'enceinte, ladite tige support étant disposée de manière à être entraînée par la rotation de chacun des deux arceaux. La cloison est également rapportée sur cette platine. On dispose ainsi d'un dispositif d'échographie compact par la platine qui assure à la fois la fixation de l'enveloppe d'enceinte et celle de la cloison. La commande de ce dispositif est également simplifié par le fait que les instructions de déplacement ne concernent que la commande des deux arceaux et des deux actionneurs associés, et la commande éventuelle en rotation propre de la tige.

L'invention concerne également l'application des caractéristiques précédemment citées à un système de télé-échographie dans lequel on réalise à distance le déplacement d'une sonde d'échographie, le dispositif de télé-échographie comportant d'une part du matériel sur un site d'expertise où un médecin qualifié peut diriger à distance l'échographie par l'intermédiaire d'une sonde fictive adaptée à transmettre des informations à un module de commande afin de piloter le déplacement des moyens capteurs, et d'autre part du matériel embarqué dans un site patient isolé où se trouve le patient à examiner, et dans lequel le site patient comporte un dispositif d'échographie tel que décrit précédemment et adapté à réaliser l'échographie sur le patient en fonction des informations de commande de déplacement des moyens capteurs qui sont calculés en fonction du déplacement de la sonde fictive sur le site d'expertise.

D'autres caractéristiques et avantages de l'invention ressortiront à la lecture de la description qui suit de ses modes de réalisation, illustrée par :
- la figure 1 qui est une illustration schématique d'un principe de télé-échographie dans lequel s'inscrit le dispositif d'échographie selon l'invention dans un mode de réalisation particulier ;
- la figure 2 qui est une vue en perspective d'un dispositif d'échographie selon l'invention dans le cadre de l'application à un système de télé-échographie illustrée sur la figure 1, les moyens capteurs étant représentés dans une position initiale, sous la forme de capteur linéaire ;

- la figure 3 qui est une vue en perspective, de dessous, du dispositif illustré à la figure 2, les moyens capteurs étant représentés dans la position initiale, sous la forme de capteur matriciel ;
- la figure 4 qui est une vue en perspective du dispositif illustré à la figure 3, les moyens capteurs étant représentés dans une première position déterminée, sous la forme de capteur matriciel ;
- la figure 5 qui est une vue de côté du dispositif de la figure 4 ;
- la figure 6 qui est une vue en perspective de dessous du dispositif illustré à la figure 2, les moyens capteurs étant ici représentés dans la première position déterminée, sous la forme de capteur linéaire ;
- et les figures 7 et 8 qui sont des vues de côté d'un dispositif d'échographie selon l'invention dans un deuxième mode de réalisation particulier, avec les moyens capteurs positionnés dans deux positions distinctes.

Dans ce qui suit, sans en limiter en quoi que ce soit la portée, on se placera ci-après dans le cadre de l'application préférée de l'invention, sauf mention contraire, c'est-à-dire dans le cas d'un système de télé-échographie, piloté à distance par un médecin qualifié.

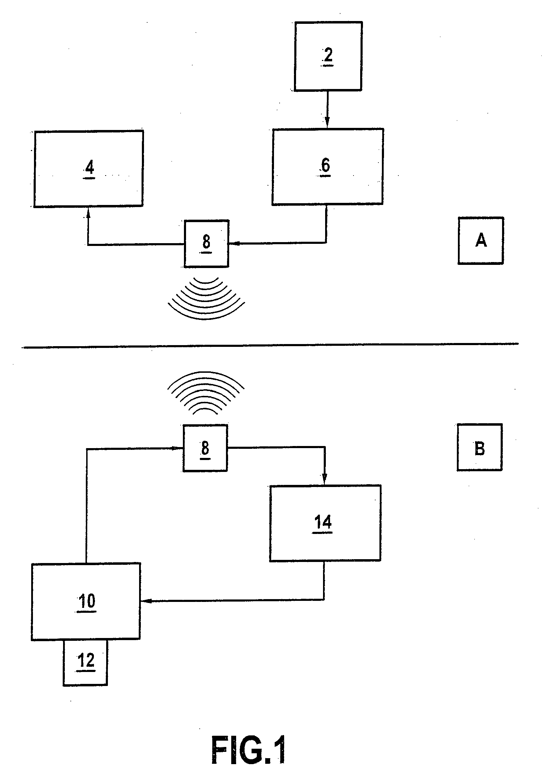

Un système de télé-échographie est représenté sur la figure 1. Il comporte d'une part du matériel sur un site A où un médecin qualifié peut diriger à distance l'échographie et d'autre part du matériel embarqué dans un site B où se trouve le patient à examiner.

Le site d'expertise A comporte une sonde fictive 2 que le médecin qualifié peut manier à sa guise en fonction des images échographiques du patient qui sont affichées sur le moniteur 4. Les déplacements de la sonde fictive 2 sont mesurés par des capteurs intégrés dans cette sonde et les valeurs mesurées sont transmises à un poste de traitement 6, qui code ces valeurs en une trame informatique de manière à pouvoir les communiquer via des émetteurs récepteurs 8 depuis le site d'expertise A vers le site patient B.

Le site patient B comporte un dispositif d'échographie télécommandé 10 adapté à réaliser l'échographie sur le patient par l'intermédiaire d'une sonde réelle 12, en fonction des instructions de commande calculées en fonction du déplacement de la sonde fictive sur le site d'expertise A.

Le résultat des mesures ultrasonores prises par la sonde réelle sur le patient permettent la réalisation d'images échographiques qui, via les émetteurs récepteurs, se retrouvent projetées sur le moniteur 4 pour que le médecin qualifié puisse visionner ces images et adapter le mouvement sur la sonde fictive pour collecter d'autres images.

Un module de commande 14 est adapté à piloter le dispositif d'échographie télécommandé pour reproduire les mouvements de la sonde fictive 2. A cet effet, le module de commande reçoit, via les émetteurs récepteurs 8, les valeurs codées représentatives du déplacement de la sonde fictive.

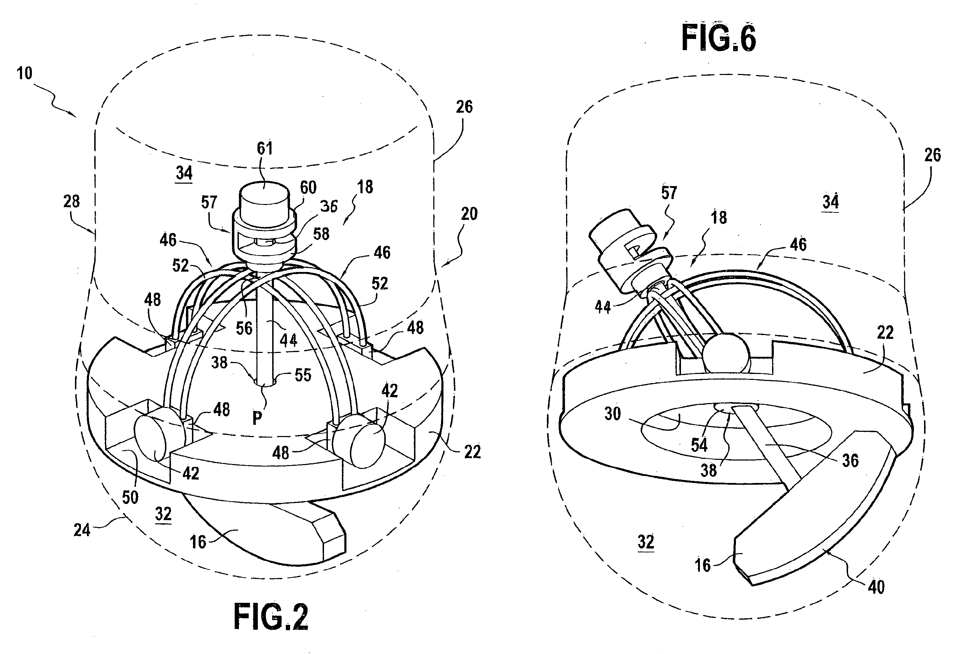

Tel qu'illustré sur la figure 2, le dispositif d'échographie 10 comporte des moyens capteurs 16 pour la prise d'images échographiques et des moyens d'entraînement 18 en déplacement de ces moyens capteurs, ainsi qu'une enveloppe d'enceinte 20 qui entoure le tout, en étant fixée sur une platine transversale 22 qui sert par ailleurs de support de fixation aux moyens d'entraînement.

L'enveloppe comporte deux coques, dont une première coque de forme hémisphérique 24 et une deuxième coque de forme cylindrique 26, qui est déformée pour former des moyens de préhension 28. L'enveloppe est formée au moins pour la coque hémisphérique dans un matériau transparent aux ondes ultrasonores.

Tel que cela est visible dans le premier mode de réalisation illustré aux figures 2 à 6, la platine porte en outre une cloison 30 qui s'étend transversalement à la paroi cylindrique de l'enveloppe d'enceinte, sensiblement au niveau de la jonction des deux coques formant l'enveloppe, de manière à délimiter à l'intérieur de l'enveloppe d'enceinte un compartiment étanche 32 et un compartiment de commande 34. Le compartiment étanche correspond à l'intérieur de la première coque hémisphérique alors que le compartiment de commande correspond à la deuxième coque cylindrique. On observe que la forme hémisphérique du compartiment étanche est déterminée par la cloison qui en forme la base et par le point fixe P au centre de la cloison qui forme le centre de la sphère dont la moitié inférieure délimite le compartiment étanche

On observe que les moyens capteurs sont logés dans le compartiment étanche tandis que les moyens d'entraînement sont logés dans le compartiment de commande. Une tige support 36 est intercalée entre les moyens capteurs et les moyens d'entraînement en s'étendant axialement et notamment à travers la cloison. A cet effet, la cloison présente en son centre un passage axial 38. La tige support traverse la cloison au niveau du passage axial de sorte qu'elle permet la mise en mouvement des moyens capteurs par l'actionnement préalable des moyens d'entraînement, en s'assurant que les moyens capteurs restent disposés dans le compartiment étanche tandis que les moyens d'entraînement restent dans le compartiment de commande.

Les moyens capteurs sont montés à l'extrémité distale de la tige support. Ils présentent une face de mesure 40 qui est opposée à la tige support et qui est tournée vers la surface de l'enveloppe. Cette face de mesure présente un rayon de courbure donné, légèrement inférieur au rayon de courbure de l'enveloppe d'enceinte. De la sorte, les moyens capteurs sont adaptés à se déplacer en surface le long de l'enveloppe d'enceinte dès lors qu'ils tournent autour du même point fixe que le point P défini précédemment comme le centre de la sphère correspondant à la forme hémisphérique du compartiment étanche.

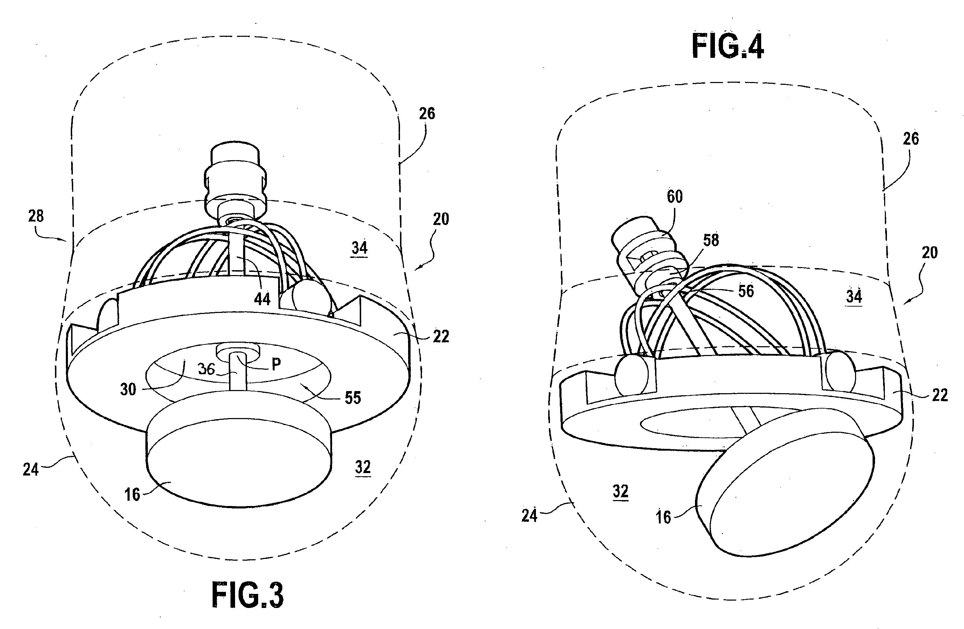

Sur la face de mesure sont disposés des composants connus et nécessaires à la transmission et la réception d'ondes ultrasonores pour la réalisation d'images échographiques. Des transducteurs piézoélectriques sont ainsi arrangés linéairement, en formant une barrette de détection (visible sur les figures 2 et 6), ou dans deux dimensions sur la face de mesure en formant une matrice de détection (visible sur les figures 3 et 4), et ils sont mis en fonctionnement à tour de rôle d'après des instructions en provenance du module de commande.

Il convient de laisser un jeu entre la face de mesure des moyens capteurs et la surface de l'enveloppe d'enceinte pour que les moyens capteurs puissent se déplacer à l'intérieur de l'enveloppe fixe. Ce jeu de fonctionnement, même s'il est minime, peut empêcher la propagation des ondes depuis et vers les moyens capteurs si de l'air circule entre les moyens capteurs et l'enveloppe d'enceinte. De la sorte, dans le compartiment étanche du dispositif d'échographie-télécommandé, qui s'étend sous la cloison, et dans ce mode de réalisation, sous la platine, les moyens capteurs sont immergés dans un milieu propagateur d'ondes ultrasonores, ici un bain d'huile. Ce milieu permet la propagation des ondes ultrasonores émises et reçues par les moyens capteurs.

La cloison est prévue étanche, ici à l'huile, pour que le milieu propagateur d'ondes reste confiné dans le compartiment étanche. Ainsi, les moyens d'entraînement qui sont logés intégralement dans la partie de commande sont protégés de l'huile par la cloison.

Comme décrit précédemment, les moyens d'entraînement visent à entraîner en déplacement les moyens capteurs par l'intermédiaire de la tige support. Les moyens d'entraînement comportent des actionneurs 42 commandés par des instructions en provenance du module de commande 14, des éléments articulés pilotés par les actionneurs et un tube de guidage 44 relié à une première extrémité aux éléments articulés et à une deuxième extrémité à la cloison, et à l'intérieur duquel est logée la tige support.

Dans le premier mode de réalisation, illustré sur les figures 2 à 6, les éléments articulés comportent deux arceaux 46 montés respectivement en pivotement autour d'un axe porté par la platine. Ces arceaux sont disposés sur la platine de telle sorte que leurs axes de pivotement respectifs soient perpendiculaires entre eux.

Les arceaux présentent une forme globale de demi-cercle dont les extrémités diamétralement opposées sont reliées chacune à un caisson 48 qui est monté à rotation sur la platine. Pour chacun des arceaux, un des caissons est associé en prise directe à un actionneur rotatif 42 qui permet, selon des instructions en provenance du module de commande, d'entraîner l'arceau en pivotement autour de l'axe de pivot commun à ses deux caissons. Les actionneurs et les caissons sont logés dans des empreintes 50 formées sur la platine transversale et diamétralement opposées deux à deux.

Les arceaux sont formés respectivement de deux fils courbes 52 qui s'étendent parallèlement à distance l'un de l'autre pour former un chemin pour le tube de guidage 44.

L'entraxe entre les fils d'un même arceau est très légèrement supérieur au diamètre du tube de guidage de sorte que celui-ci est directement en contact avec les arceaux. Pour un arceau donné, le tube est bloqué latéralement par les fils de cet arceau mais il peut glisser axialement le long du chemin de guidage.

L'agencement du tube et des arceaux est tel que le pivotement d'un arceau entraîne en déplacement le tube qui est bloqué par les fils de cet arceau, le tube étant alors adapté à glisser le long du chemin de guidage de l'autre arceau.

L'extrémité du tube opposée aux moyens d'entraînement est rendue solidaire d'un joint d'étanchéité 54, à titre d'exemple un joint tournant, emmanché au centre de la cloison dans le passage axial, de sorte que le pivotement des arceaux entraîne le basculement du tube de guidage autour du point de jonction du tube au centre de la cloison, qui se trouve être le point fixe P défini précédemment. On prévoit sur chacune des faces de la platine, tel que cela est visible sur les figures 2 et 3 par exemple, des alésages 55 de plus grand diamètre que le diamètre extérieur du joint d'étanchéité, de manière à permettre l'inclinaison du tube par rapport à la verticale lorsqu'il pivote.

Dans les modes de réalisation illustrés, on observe que le tube ne traverse pas la cloison, son extrémité étant rendue solidaire de la cloison via le joint d'étanchéité. On crée ainsi au centre de la cloison un point de pivot du tube qui reste fixe, de sorte que le tube pivote lorsque son extrémité opposée est entraînée par les éléments articulés. On comprendra qu'on peut prévoir un tube traversant la cloison en conservant ce point fixe P au centre de la cloison autour duquel le tube bascule.

C'est donc le pivotement des arceaux autour de leur axe respectif qui génère le déplacement du tube de guidage par rapport au point fixe P, qui est situé au croisement des axes de pivot perpendiculaires des arceaux, et qui est le centre de la sphère délimitant la forme hémisphérique du compartiment étanche. Comme cela sera décrit ci-après, on peut prévoir un pivotement simultané des deux arceaux ou bien seulement de l'un ou l'autre. Comme illustré sur les figures, le premier arceau présente un diamètre à sa base plus grand que celui du deuxième arceau, et les empreintes permettant de loger les caissons et l'actionneur associés au premier arceau sont plus écartées l'une de l'autre que celles permettant de loger les caissons et l'actionneur associés au deuxième arceau. On s'assure ainsi de cette façon que l'un des arceaux passe au-dessus de l'autre, mais on comprendra qu'on pourra réaliser ceci par d'autres moyens équivalents. L'essentiel est que le pivotement de chacun des deux arceaux est rendu possible par le fait qu'un premier arceau soit dimensionné de sorte qu'il puisse passer au-dessus de l'autre. On crée ainsi une zone carrée de recouvrement 56 délimitée par quatre parties de fils parallèles deux à deux à l'intérieur de laquelle le tube est pris en tenaille. Le pivotement d'un arceau génère le déplacement du tube par rapport à un axe perpendiculaire à l'axe de pivotement, et on comprend que la combinaison des pivotements des deux arceaux permet le déplacement du tube selon plusieurs axes.

Comme illustré sur les figures, la tige support des moyens capteurs est logée à l'intérieur du tube de sorte qu'elle suit les déplacements de ce tube et donc les pivotements de l'un et de l'autre des arceaux. Les moyens capteurs situés en bout de la tige support sont ainsi entraînés selon un déplacement symétrique à celui du tube par rapport au point de passage. Avantageusement, la tige support est montée libre en rotation à l'intérieur du tube, de sorte que la tige support peut être entraînée en rotation sur elle-même sans que le tube ne tourne. Comme cela sera indiqué ci-après, ce montage présente l'avantage de ne pas détériorer la cloison et le joint associé au centre desquels le tube et la tige support sont disposés.

A cet effet, l'extrémité du tube disposée dans la partie de commande est munie d'une bague 57 fixée sur le tube. La bague s'étend de l'autre côté des arceaux par rapport à la cloison, et elle comporte une embase 58, de diamètre plus grand que l'entraxe entre les fils d'un arceau, et une collerette 60 sur laquelle est rapporté un troisième actionneur 61 en prise directe avec la tige support logée à l'intérieur du tube, le troisième actionneur étant adapté à recevoir des instructions de commande pour la mise en rotation de la tige sur son axe propre. La liaison de la tige avec le troisième actionneur, qui est figé sur la bague qui s'étend au-delà des arceaux et dont l'embase repose sur les fils de l'arceau supérieur, offre une butée en translation selon l'axe de la tige support pour éviter que cette dernière ne s'échappe et que les moyens capteurs se retrouvent plaqués contre l'enveloppe d'enceinte sans jeu fonctionnel.

La butée en sens inverse est assurée par la fixation du tube sur la cloison et par la fixation de l'actionneur sur la bague solidaire du tube. Si l'on venait à renverser le dispositif, l'actionneur resterait mécaniquement à la distance déterminée et renseignée dans le module de commande et la tige support en prise directe sur l'actionneur ne bougerait pas non plus.

Le module de commande 14 du dispositif est adapté à fournir des signaux de commande à destination de chacun de ces actionneurs afin de commander le déplacement en rotation des moyens capteurs en conformité avec les instructions de rotation sur la sonde fictive manipulée à distance par le médecin qualifié. Le logiciel intégré dans le module de commande est paramétré pour transformer le pivotement autour d'un point donné de la sonde fictive en des coordonnées de déplacement des moyens capteurs et d'allumage des transducteurs de ces mêmes moyens capteurs. On comprendra que le logiciel pourra être modifié pour adapter la transformation à d'autres moyens d'entraînement ou d'autres types de capteurs.

Par ailleurs, chaque actionneur est associé à un capteur de position angulaire non représenté qui permet de déterminer la position angulaire absolue des arceaux et de la tige support et donc la position absolue des moyens capteurs dans le compartiment étanche.

On observe que, pour des raisons pratiques de lecture des figures 2 à 6, les câbles permettant la transmission des signaux de commande entre les actionneurs, les moyens capteurs et le module de commande, de même que les câbles d'alimentation, n'ont pas été représentés. Dans le dispositif selon l'invention, la présence de ces câbles n'impacte pas l'étanchéité de l'ensemble. Les câbles reliant les moyens capteurs au module de commande peuvent s'étendre à l'intérieur du tube ou de la tige ou bien ils peuvent passer à travers un presse-étoupe disposé de façon étanche au niveau de la cloison, de sorte que dans tous les cas, le passage de ces câbles ne gêne pas l'étanchéité de la cloison alors que l'ensemble des actionneurs est situé dans le compartiment de commande. Les câbles reliant ces actionneurs pourront avantageusement être repris sur la platine pour les faire remonter le long de l'enveloppe.

On comprend de ce qui précède que les moyens capteurs sont montés de manière à se déplacer de façon pendulaire par rapport à un point fixe déterminé pour qu'ils réalisent un déplacement surfacique, à distance constante de la paroi sphérique du compartiment étanche. Selon l'invention, ce point fixe est disposé au centre de la cloison et cela est particulièrement avantageux en ce que les mouvements de rotation de la tige support et des moyens capteurs n'entraînent ainsi pas de frottements et de contraintes sur la cloison qui ne risque pas de se déchirer et qui peut donc assurer sa fonction d'étanchéité dans le temps. On prévoit avantageusement que la tige support soit logée à l'intérieur d'un tube centré sur la cloison pour permettre la rotation propre des moyens capteurs sans entraîner là non plus de frottements et de contraintes sur la cloison.

Dès lors, l'invention est particulièrement intéressante en ce qu'elle propose un dispositif d'échographie télécommandé pour des opérations à distance, et notamment pour la télé-échographie dans le domaine médical, dans lequel des moyens capteurs sont rendus mobile à l'intérieur d'une enveloppe d'enceinte qui reste fixe et qui recouvre l'ensemble du dispositif appliqué sur le corps du patient. On protège les moyens capteurs qui dans le contexte d'utilisation vont être manipulés par du personnel non qualifié et on rend plus confortable l'opération pour le patient puisque l'opérateur ne sait pas avec quelle force il doit appuyer sur le ventre du patient et il a tendance à trop appuyer ce qui crée des pincements du ventre lorsque la sonde pivote.

Les moyens d'entraînement permettent la mise en mouvement des moyens capteurs, ce qui a pour effet de limiter la taille de ces moyens capteurs. On constate avantageusement que le fait de diminuer la taille des moyens capteurs améliore l'image échographique puisque l'on peut alors augmenter la fréquence de prise d'image. Par ailleurs, le fait de combiner aux mouvements mécaniques un balayage électronique des moyens capteurs permet de diminuer les dimensions mécaniques des moyens d'entraînement.

Il en résulte que l'on peut s'adapter à la taille et au rayon de courbure des capteurs pour réaliser un dispositif d'échographie le plus compact possible. Et le fait d'avoir des actionneurs en prise directe avec les éléments articulés à actionner va dans le sens de la compacité recherchée.

Cette compacité du dispositif permet de plaquer la coque hémisphérique sur le corps du patient dans des zones peu accessibles de sorte que l'on peut réaliser des images échographiques de zones jusqu'alors irréalisables à cause de l'encombrement du support de la sonde réelle. De même il est dès lors facile d'embarquer le dispositif pour des utilisations dans des lieux spécifiques et par exemple pour une utilisation dans les voyages spatiaux.

On va maintenant décrire l'utilisation du dispositif d'échographie selon l'invention dans des examens de télé-échographie.

Sur le site patient, on positionne la sonde réelle de telle sorte que la coque hémisphérique de l'enveloppe d'enceinte soit au contact de la zone de travail, ici le corps du patient, en regard de la zone à examiner. Les images échographiques prises par la sonde sont envoyées via les émetteurs récepteurs vers le moniteur présent sur le site d'expertise et ces images sont analysées par le médecin qualifié. Afin de parfaire son analyse, le médecin qualifié cherche à obtenir d'autres images en inclinant sa sonde fictive, et ces mouvements sont reproduits sur le site patient via le dispositif robotisé.

Les mouvements de rotation de la sonde fictive sont traduits et transmis au module de commande via le poste de traitement et les émetteurs récepteurs comme cela a été décrit précédemment par la création d'une trame informatique à base d'octets représentative de ces mouvements. Le logiciel intégré du module de commande détermine alors par le calcul quels doivent être les mouvements des moyens d'entraînement et de la tige support pour reproduire fidèlement le déplacement de la sonde fictive, et des signaux de commande correspondants sont élaborés pour chacun des actionneurs du dispositif d'échographie.

Le logiciel calcule dans un premier temps le point de coordonnées sur la surface de l'enveloppe en regard duquel doit être placé le centre des moyens capteurs et l'angle de rotation propre. Il calcule dans un deuxième temps, par rapport à une position initiale précédente des moyens articulés et des moyens capteurs, le pivotement de chaque arceau autour de son axe respectif qu'il faut réaliser pour amener le centre des moyens capteurs en le point de coordonnées déterminé auparavant.

Ainsi, on comprend que pour passer de la position initiale illustrée à la figure 3 à la position intermédiaire illustrée à la figure 4, le module de commande a reçu des informations en provenance du site d'expertise relatives à l'inclinaison de la sonde fictive par le médecin qualifié. Le logiciel intégré a calculé sur la base de ces informations une instruction de commande pour l'actionneur associé au premier arceau, ici l'arceau supérieur de plus grand diamètre, une instruction de commande pour l'actionneur associé au deuxième arceau, ainsi qu'une instruction pour le troisième actionneur en prise directe avec la tige support et une instruction de déclenchement des transducteurs des moyens capteurs. Pour arriver ici à la position intermédiaire, l'actionneur associé au premier arceau a été mis en rotation alors que l'actionneur associé au deuxième arceau est resté inactif. Le tube s'est déplacé le long du chemin de guidage du deuxième arceau parallèlement à l'axe de rotation de ce deuxième arceau.

On comprendra que le logiciel intégré dans le module de commande, qui est adapté à transformer le mouvement de la sonde fictive en des signaux de commande du dispositif d'échographie à la fois pour les actionneurs et pour les moyens capteurs, est d'un type connu et qu'il pourra être paramétré différemment pour s'adapter notamment à des agencements particuliers des moyens d'entraînement. Dans le cas présent, le module de commande reçoit en entrée les valeurs d'encombrement du dispositif embarqué sur le site patient et notamment la distance entre la bague de guidage portée à une extrémité du tube et le centre des moyens capteurs, le rayon des arceaux et le rayon de l'hémisphère le long de laquelle doivent se déplacer surfaciquement les moyens capteurs. Et le logiciel est adapté à calculer les instructions de commande en fonction de ces données d'entrée et de la trame représentative des déplacements de la sonde fictive qu'il reçoit du poste de traitement.

En temps réel, le module de commande reçoit d'une part les instructions de déplacement de la sonde réelle, pour les transmettre en autant de signaux de commande que nécessite le cas d'espèce et il reçoit d'autre part des informations en provenance des actionneurs et des capteurs de position angulaire de manière à avoir en temps réel une donnée fiable quant au positionnement des moyens d'entraînement, afin de pouvoir calculer les signaux de commande adéquats par la suite pour piloter l'ensemble en accord avec les instructions du médecin qualifié.

Les mouvements opérés par le médecin qualifié sur la sonde fictive de pilotage sont ainsi réalisés à distance par la sonde réelle qui reproduit l'orientation de la sonde fictive directement sur le corps du patient et permet la prise de vue d'images échographiques.

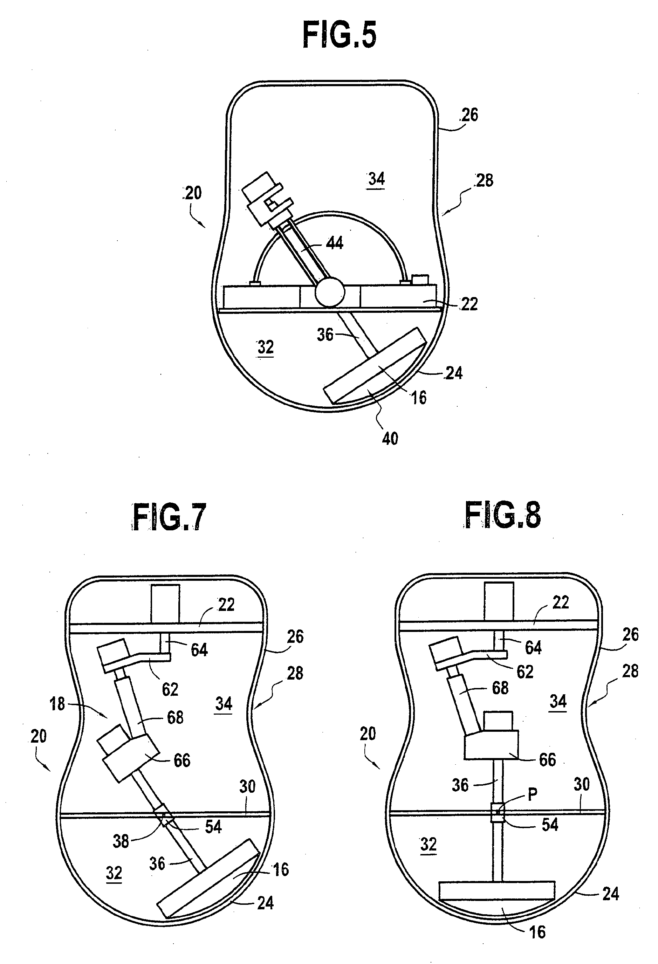

On va maintenant décrire un deuxième mode de réalisation tel qu'il est illustré aux figures 7 et 8. Le dispositif d'échographie selon ce mode de réalisation diffère de celui décrit précédemment en ce que les moyens d'entraînement sont formés par un ensemble de bras articulés au lieu d'un dispositif d'arceaux. Il en résulte que la platine est disposée en partie haute de l'enveloppe d'enceinte pour supporter les contraintes mécaniques des bras et des actionneurs. La cloison s'étend elle sensiblement à la même hauteur que précédemment mais il convient de prévoir des moyens de fixation propre de cette cloison par rapport à l'enveloppe d'enceinte.

L'ensemble de bras articulés comporte un bras supérieur 62 qui est solidaire en rotation d'un premier arbre 64 monté pivotant sur la platine, et il comporte un bras intermédiaire 66 solidaire d'un deuxième arbre de rotation 68 monté pivotant à l'extrémité libre du bras supérieur. Chaque arbre est entraîné en rotation par un actionneur associé. La tige support des moyens capteurs est montée à l'extrémité libre du bras intermédiaire. Comme précédemment, la tige support est adaptée à être entraîné en rotation propre. On prévoit un agencement des bras et des arbres pour que les moyens capteurs tournent autour d'un point fixe disposé au centre de cette cloison. La tige support des moyens capteurs passe au centre de la cloison et est montée libre en rotation propre dans un tube passant au centre de la cloison.

Là encore, le module de commande est adapté à fournir des signaux de commande à destination des actionneurs associés respectivement à chacun des éléments des moyens d'entraînement, c'est-à-dire ici à chacun des bras articulés, afin de commander le déplacement des moyens capteurs.

Et, de même que dans le premier mode de réalisation, chaque actionneur est associé à un capteur de position angulaire qui permet de déterminer la position angulaire absolue des bras articulés et donc la position des moyens capteurs dans le compartiment étanche.

On comprendra que l'invention n'est pas limitée aux seuls dispositifs conformes aux modes de réalisation explicitement décrits en regard des figures 1 à 8, et il convient également de noter que l'invention n'est pas limitée à l'application préférée relative à des opérations de télé-échographie dans le domaine médical.

## Revendications

1. Dispositif d'échographie télécommandé comportant une enveloppe d'enceinte (20) à l'intérieur de laquelle sont disposés un compartiment étanche (32), dans lequel des moyens capteurs (16) sont adaptés à se déplacer, immergés dans un milieu propagateur d'ondes ultrasonores, et un compartiment de commande (34) séparé de manière étanche dudit compartiment étanche par une cloison transversale (30), des moyens d'entraînement (18) étant logés dans le compartiment de commande pour la mise en mouvement d'une tige support (36) à l'extrémité de laquelle sont montés les moyens capteurs, **caractérisé en ce que** ladite tige support des moyens capteurs est montée libre à rotation à l'intérieur d'un tube (44) passant à travers ladite cloison, en un point fixe (P) disposé au centre de la cloison, ledit tube (44) étant monté basculant par rapport audit point fixe (P), de sorte que les moyens capteurs (16) sont adaptés à se déplacer dans ledit compartiment étanche (32) de façon pendulaire par rapport à ce point fixe, un joint d'étanchéité (54) disposé autour du tube étant plaqué contre la cloison, et **en ce qu'**il est prévu un actionneur pour entraîner ladite tige support des moyens capteurs en rotation propre.

2. Dispositif selon la revendication 1, **caractérisé en ce que** le tube (44) porte à son extrémité opposée aux moyens capteurs une bague (57) qui s'étend au-delà des moyens d'entraînement (18) et qui porte ledit actionneur (61) en prise directe sur la tige support (36) montée libre à rotation à l'intérieur du tube.

3. Dispositif selon l'une des revendications précédentes, **caractérisé en ce que** le compartiment étanche (32) présente une forme hémisphérique dont la base est formée par ladite cloison (30) et dont le centre est ledit point fixe (P) au centre de ladite cloison, et **en ce que** les moyens capteurs (16) présentent un rayon de courbure légèrement inférieur au rayon de courbure du compartiment étanche, de manière à assurer un déplacement surfacique des moyens capteurs le long de la surface du compartiment étanche.

4. Dispositif selon l'une des revendications précédentes, **caractérisé en ce qu'**il est associé à un module de commande (14) et qu'il est adapté à recevoir dudit module de commande des instructions de fonctionnement des moyens d'entraînement (18) dans le compartiment de commande (34) pour le déplacement des moyens capteurs (16) dans le compartiment étanche (32) ainsi que des informations d'activation des éléments piézoélectriques constitutifs des moyens capteurs.

5. Dispositif selon l'une des revendications précédentes, **caractérisé en ce que** les moyens d'entraînement (18) comportent deux arceaux (46) disposés perpendiculairement et montés à pivotement sur une platine (22) sur laquelle est fixée l'enveloppe d'enceinte (20), ladite tige support (36) étant disposée de manière à être entraînée par le pivotement de chacun des deux arceaux.

6. Dispositif selon la revendication 5, **caractérisé en ce que** la cloison (30) est rapportée sur ladite platine (22).

7. Dispositif selon l'une des revendications 1 à 4, **caractérisé en ce que** les moyens d'entraînement (18) sont formés par un ensemble de bras articulés qui comporte un bras supérieur (62) solidaire en rotation d'un premier arbre (64) monté pivotant sur ladite platine, un bras intermédiaire (66) solidaire d'un deuxième arbre de rotation (68) monté pivotant à l'extrémité libre du bras supérieur, ladite tige support des moyens capteurs (36) étant montée à l'extrémité libre du bras intermédiaire, lesdits bras et lesdits arbres étant agencés pour que les moyens capteurs (16) en bout de la tige support se déplacent de façon pendulaire autour d'un point fixe (P) disposé au centre de ladite cloison (30) rapportée sur l'enveloppe d'enceinte (20) à distance d'une platine (22) sur laquelle sont fixés l'enveloppe d'enceinte et les moyens d'entraînement.

8. Ensemble de télé-échographie dans lequel une sonde d'échographie (12) est déplacée à distance et qui comporte, d'une part, du matériel sur un site d'expertise (A) où un médecin qualifié peut diriger à distance l'échographie et, d'autre part, du matériel embarqué dans un site patient (B) où peut se trouver le patient à examiner, **caractérisé en ce que** le site patient (B) comporte un dispositif d'échographie télécommandé selon l'une des revendications précédentes, adapté à réaliser l'échographie sur le patient en fonction des informations de commande qui découlent du déplacement d'une sonde fictive (2) sur le site d'expertise (A).

## Patentansprüche

1. Vorrichtung zur ferngesteuerten Ultraschalluntersuchung, aufweisend eine Einfassungshülle (20), in deren Inneren Folgendes angeordnet ist: eine dichte Kammer (32), in der in ein Ultraschallwellen-Ausbreitungsmedium eingetauchte Sensormittel (16) so angebracht sind, dass sie verschiebbar sind, und eine von der dichten Kammer durch eine querlaufende Trennwand (30) hermetisch abgetrennte Steuer-Kammer (34), Antriebsmittel (18), die in der Steuerkammer untergebracht sind, um einen Trägerschaft (36) in Bewegung zu versetzen, an dessen Ende die Sensormittel montiert sind, **dadurch gekennzeichnet, dass** der Trägerschaft der Sensoreinrichtungen frei drehbar im Inneren eines Rohres (44), welches quer zu der Trennwand verläuft, an einem in der Mitte der Trennwand angeordneten Fixpunkt (P) montiert ist, wobei das Rohr (44) kippbar bezüglich des Fixpunkts (P) so montiert ist, dass die Sensormittel (16) in der Lage sind, sich in der Dichtungskammer (32) senkrecht bezüglich dieses Fixpunkts zu verschieben, wobei eine Abdichtung (54), die um das Rohr angeordnet ist, an der Trennwand anliegt, und dadurch, dass ein Betätigungselement zum Antreiben des Trägerschafts der Sensormittel zur Eigenrotation vorgesehen ist.

2. Vorrichtung nach Anspruch 1, **dadurch gekennzeichnet, dass** das Rohr (44) an seinem den Sensormitteln gegenüberliegenden Ende einen Ring (57) trägt, der sich über die Antriebsmittel (18) hinaus erstreckt und der das Betätigungselement (61) in direktem Eingriff auf dem im Inneren des Rohres frei drehbar montierten Trägerschaft (36) trägt.

3. Vorrichtung nach einem der vorhergehenden Ansprüche, **dadurch gekennzeichnet, dass** die dichte Kammer (32) eine halbkugelige Form aufweist, deren Basis durch die Trennwand (30) gebildet ist und deren Zentrum der Fixpunkt (P) in der Mitte der Trennwand ist, und dadurch dass die Sensormittel (16) einen Krümmungsradius aufweisen, der etwas kleiner ist als der Krümmungsradius der dichten Kammer, derart, dass eine Oberflächenverschiebung der Sensoreinrichtungen entlang der Oberfläche der dichten Kammer sichergestellt ist.

4. Vorrichtung nach einem der vorhergehenden Ansprüche, **dadurch gekennzeichnet, dass** sie einem Steuermodul (14) zugehörig ist und dass sie dazu ausgelegt ist, von dem Steuermodul Funktionsanweisungen der Antriebsmittel (18) in der Steuerkammer (34) zur Verschiebung der Sensoreinrichtungen (16) in der dichten Kammer (32) sowie Aktivierungsinformationen der piezoelektrischen Elemente zu empfangen, aus denen die Sensormittel bestehen.

5. Vorrichtung nach einem der vorhergehenden Ansprüche, **dadurch gekennzeichnet, dass** die Antriebsmittel (18) zwei Bügel (46) aufweisen, die senkrecht angeordnet und auf einer Platte (22) schwenkbar gelagert sind, auf der die Einfassungshülle (20) fixiert ist, wobei der Trägerschaft (36) so angeordnet ist, dass er durch das Schwenken eines jeden der beiden Bügel angetrieben wird.

6. Vorrichtung nach Anspruch 5, **dadurch gekennzeichnet, dass** die Trennwand (30) auf der Platte (22) angebracht ist.

7. Vorrichtung nach einem der Ansprüche 1 bis 4, **dadurch gekennzeichnet, dass** die Antriebsmittel (18) durch einen Satz von Gelenkarmen gebildet sind, der einen oberen mit einer ersten Welle (64) drehfesten Arm (62), der schwenkbar auf der Platte montiert ist, einen Zwischenarm (66), der mit einer Drehwelle (68) fest verbunden ist, die am freien Ende des oberen Arms schwenkt, aufweist, wobei der Trägerschaft der Sensormittel (36) am freien Ende des Zwischenarms montiert ist, wobei die Arme und die Wellen so angeordnet sind, dass sich die Sensoreinrichtungen (16) am Ende des Trägerschafts senkrecht um einen Fixpunkt (P) verschieben, der in der Mitte der Trennwand (30) angeordnet ist, die an die Einfassungshülle (20) von einer Platte (22) beabstandet angefügt ist, auf der die Einfassungshülle und die Antriebsmittel befestigt sind.

8. Ultraschallanordnung, bei der sich eine Ultraschallwelle (12) über eine Strecke ausbreitet, und die einerseits Gerätschaft an einem Begutachtungsort
(A) aufweist, wo ein qualifizierter Arzt die Ultraschalluntersuchung fernsteuern kann, und andererseits Gerätschaft aufweist, die sich an einem Patientenort
(B) befindet, wo sich der zu untersuchende Patient befinden kann, **dadurch gekennzeichnet, dass** der Patientenort (B) eine Ultraschallvorrichtung nach einem der vorangehenden Ansprüche aufweist, die in der Lage ist, die Ultraschalluntersuchung an einem Patienten in Abhängigkeit der Steuerinformationen vorzunehmen, die sich aus der Verschiebung einer fiktiven Sonde (2) an dem Begutachtungsort (A) ergeben.

## Claims

1. A remote-controlled ultrasound device comprising an outer enclosure (20) inside which there are a sealed compartment (32), in which sensor means (16) are designed to move, these being immersed in a medium through which ultrasonic waves can propagate, and a control compartment (34) which is separated in a sealed manner from said sealed compartment by a transverse partition (30), drive means (18) being housed in the control compartment for setting in motion a support rod (36) at the end of which are mounted the sensor means, **characterised in that** said support rod of the sensor means is mounted free to rotate inside a tube (44) passing through said partition, at a fixed point (P) located at the centre of the partition, said tube (44) being mounted so that it can swing relative to the fixed point (P), such that the sensor means (16) are able to move in said sealed compartment (32) in a pendular manner relative to this fixed point, a seal (54) arranged around the tube being pressed firmly against the partition, and **in that** there is provided an actuator for driving said support rod of the sensor means rotationally about itself.

2. Device according to Claim 1, **characterised in that** tube (44) has, at its end opposite to the sensor means, a ring (57) which extends beyond the drive means (18) and which bears said actuator (61) in direct contact with the support rod (36) mounted free to rotate inside the tube.

3. Device according any one of the preceding claims, **characterised in that** sealed compartment (32) has a hemispherical shape the base of which is formed by said partition (30) and the centre of which is said fixed point (P) at the centre of said partition, and **in that** the sensor means (16) have a radius of curvature slightly smaller than the radius of curvature of the sealed compartment, so as to ensure surface movement of the sensor means along the surface of the sealed compartment.

4. Device according to any one of the preceding claims, **characterised in that** it is associated with a control module (14) and that it is able to receive, from said control module, instructions for operating the drive means (18) in the control compartment (34) for the movement of the sensor means (16) in the sealed compartment (32) as well as information regarding the activation of the piezoelectric elements that the sensor means include.

5. Device according to any one of the preceding claims, **characterised in that** the drive means (18) include two arches (46) arranged perpendicularly and mounted with pivoting on a mounting plate (22) on which is fixed the outer enclosure (20), said support rod (36) being arranged in such a way as to be driven by the pivoting of each of the two arches.

6. Device according to Claim 5, **characterised in that** the partition (30) is attached to said mounting plate (22).

7. Device according to any one of Claims 1 to 4, **characterised in that** the drive means (18) are formed by a set of articulated arms which comprises an upper arm (62) rotationally fixed to a first shaft (64) pivot-mounted on said mounting plate, an intermediate arm (66) secured to a second rotation shaft (68) pivot-mounted at the free end of the upper arm, said sensor means support rod (36) being mounted at the free end of the intermediate arm, said arms and said shafts being arranged in such a way that the sensor means (16) at the end of the support rod move in a pendular manner about a fixed point (P) positioned at the centre of said partition (30) attached to the outer enclosure (20) some distance away from a mounting plate (22) on which are fixed the outer enclosure and the drive means.

8. An ultrasound assembly in which an ultrasound probe (12) is moved remotely and which comprises, on the one hand, equipment on an expert site (A) where a qualified doctor can remotely direct the ultrasonography and, on the other hand, equipment contained at a patient site (B) where the patient to be examined is situated, **characterised in that** the patient site (B) comprises a remote-controlled ultrasound device according to any one of the preceding claims, designed for performing ultrasonography on the patient according to the control information arising from the movement of a dummy probe (2) at the expert site (A).
